# EUROPEAN PATENT APPLICATION

(11) **EP 4 005 787 A1**
(43) Date of publication of application: **01.06.2022**
(21) Application number: 20843313.6
(22) Date of filing: 20.07.2020
(51) Int. Cl.: B32B 5/02, A61Q 19/00, A61K 9/70, A61K 8/02, A61K 47/34, A61K 47/38, D06Q 1/12

(54) **TRANSFER SHEET**

(30) Priority: 24.07.2019 JP 2019136242
(71) Applicant: TOPPAN INC., Tokyo 110-0016 (JP)
(72) Inventor: TAKASHIMA, Yuichiro, Tokyo 110-0016 (JP); NAGASHIMA, Kenta, Tokyo 110-0016 (JP); ISHIKAWA, Hironori, Tokyo 110-0016 (JP); YOKOI, Hiromi, Tokyo 110-0016 (JP)
(74) Representative: TBK
(86) International application number: PCT/JP2020/028075
(87) International publication number: WO 2021/015167

(57) **Abstract**

A transfer sheet comprises a thin-film layer having an average mass of 0.01 g/m² or more and 10 g/m² or less, and a support layer that supports the thin-film layer. The support layer has a support surface in contact with the thin-film layer, and the support surface includes a plurality of fibers. The thin-film layer includes a plurality of contact parts in contact with the fibers and a non-contact part separated from the fibers. The plurality of contact parts include a contact part that is in contact with the fiber such that the contact part extends along a surface of the fiber, and whose average film thickness of the thin-film layer is less than an average film thickness of the thin-film layer in the non-contact part.

## Description

### [Technical Field]

The present disclosure relates to a transfer sheet for transferring a thin-film layer.

### [Background Art]

A thin-film layer with a thickness of around several nm to several µm adheres to the surface of a living organ. Therefore, it has been proposed to attach such thin-film layers to organs, skin, or the like for medical or cosmetic purposes.

A transfer sheet used to transfer a thin-film layer to a transfer-receiving object includes the thin-film layer and a support layer for supporting the thin-film layer. The thin-film layer is transferred to the transfer-receiving object by peeling off the support layer from the thin-film layer after bringing the surface of the thin-film layer on the support layer into contact with the object (for example, see PTL 1).

### [Citation List]

### [Patent Literature]

[PTL 1] JP 2017-19116 A

### [Summary of the Invention]

### [Technical Problem]

To transfer the thin-film layer, it is necessary to move the transfer sheet to a part of the transfer-receiving object where the thin-film layer is to be attached, and to bring the surface of the thin-film layer into contact with the target part and then peel off the support layer from the thin-film layer. For easy handling of the transfer sheet, it is preferred that movement of the transfer sheet and peeling off of the support layer can be performed simply. However, as the thin-film layer is extremely thin, if the adhesion between the thin-film layer and the support layer is too weak, when the thin-film layer is moved to the target part, the thin-film layer may peel off from the support layer due to airflow, or wrinkle or twist of the thin-film layer due to the influence of static electricity. On the other hand, if the adhesion force between the thin-film layer and the support layer is excessively high, it is difficult to peel off the support layer from the thin-film layer.

An object of the present disclosure is to provide a transfer sheet that can be handled easily.

### [Means for Solving the Problem]

A transfer sheet that solves the above-described problems includes: a thin-film layer having an average mass of 0.01 g/m² or more and 10 g/m² or less; and a support layer that supports the thin-film layer. The support layer has a support surface in contact with the thin-film layer and the support surface includes a plurality of fibers. The thin-film layer includes a plurality of contact parts in contact with the fibers and a non-contact part separated from the fibers, and the plurality of contact parts include at least one of the contact parts that is in contact with at least one of the fibers such that the at least one of the contact parts extends along a surface of the at least one of the fibers in contact therewith. The average film thickness of the thin-film layer at the at least one of the contact parts is less than the average film thickness of the thin-film layer at the non-contact part.

According to this configuration, since the thin-film layer is partially in contact with the support layer, the force of adhesion between the thin-film layer and the support layer is smaller as compared with the case where the surface of the thin-film layer is entirely in contact with the support layer. As a result, the support layer can be easily peeled off from the thin-film layer after bringing the transfer sheet into contact with the transfer-receiving object. On the other hand, at least a part of the plurality of contact parts is in contact with the fibers in such a manner that the thin-film layer spreads thinly along the surface of the fiber of the support layer. Therefore, as compared with a case where the contact parts of the thin-film layer are in point contact with the fibers, the adhesion is higher between the thin-film layer and the support. Thus, it is possible to prevent the thin-film layer from peeling off from the support layer, or wrinkling or twisting of the thin-film layer when moving the transfer sheet to the transfer-receiving object. Therefore, the transfer sheet can be easily handled.

### [Effect of the Invention]

According to the present disclosure, handling of the transfer sheet can be facilitated.

### [Brief Description of the Drawings]

Fig. 1 is a view illustrating a cross-sectional structure of a transfer sheet according to an embodiment.
Fig. 2 is a view illustrating an example of a distribution of contact parts of a transfer sheet according to an embodiment.
Fig. 3 is a view illustrating a cross-sectional structure near a contact part of a transfer sheet according to an embodiment.
Fig. 4 is an enlarged view of a cross-sectional structure near a contact part of a transfer sheet according to an embodiment.
Fig. 5 is a view illustrating another example of a cross-sectional structure near a contact part of a transfer sheet according to an embodiment.

### [Description of the Embodiments]

An embodiment of a transfer sheet will be described with reference to the drawings.

### [Overall Configuration of Transfer Sheet]

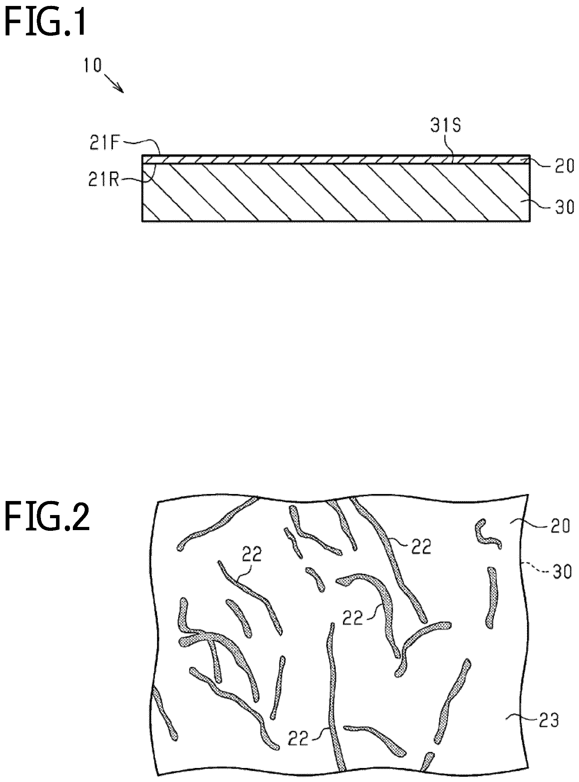

As shown in Fig. 1, the transfer sheet 10 includes a thin-film layer 20 and a support layer 30 supporting the thin-film layer 20.

The thin-film layer 20 has a first surface 21F and a second surface 21R facing away from the first surface 21F. The first surface 21F is the surface that comes into contact with the transfer-receiving object when the thin-film layer 20 is transferred. The second surface 21R is in contact with the support layer 30.

The thin-film layer 20 is thin enough to exhibit adhesiveness to the transfer-receiving object by itself. When the thinness required to exhibit adhesiveness to the transfer-receiving object is expressed in terms of the mass of the thin-film layer 20, for example, when the thin-film layer 20 has a density in the range of 1 g/cm³ or more and 3 g/cm³ or less, the average mass of the thin-film layer 20 per unit area is in the range of 0.01 g/m² or more and 10 g/m² or less. The average mass is the mass of the thin-film layer 20 per portion having an area of 1 m² in plan view. When the average mass of the thin-film layer 20 is in the above range, the thin-film layer 20 itself adheres to the surface of the transfer-receiving object such as the skin or an organ of a living organism without using an adhesive layer. Fig. 1 shows that the ratio of the thickness of the thin-film layer 20 to the thickness of the support layer 30 is larger than it actually is.

The material of the thin-film layer 20 is not particularly limited, and may be selected according to the purpose of attaching the thin-film layer 20 to the transfer-receiving object. The material of the thin-film layer 20 may be, for example, a polyester such as polylactic acid, polyglycolic acid, polycaprolactone, or polydioxanone; a polyolefin such as polyethylene or polypropylene; polyvinyl alcohol; polyamide; polyimide; polytetrafluoroethylene; polystyrene; poly(meth)acrylic acid; polyurethane; a copolymer of these polymers; a polysaccharide such as hyaluronic acid or chitosan; a protein such as casein or fibroin; silicone; a cellulose derivative such as cellulose acetate, cellulose acetate propionate, or cellulose acetate butyrate; polycarbonate; a cycloolefin copolymer; or styrene butadiene elastomer. The thin-film layer 20 may be formed of a single type of material, or a plurality of types of materials.

The thin-film layer 20 may contain a functional substance that enhances the function of the thin-film layer 20 or adds a function to the thin-film layer 20. The function substance may be, for example, a cosmetic or medicinal ingredient. When the thin-film layer 20 contains a cosmetic ingredient, a thin-film layer 20 suitable for being attached to the skin as the transfer-receiving object for cosmetic use is realized.

Examples of the cosmetic ingredients include moisturizing ingredients such as hyaluronic acid, collagen, ceramide, pullulan, and sacran, whitening ingredients such as vitamin C and its derivatives, kojic acid, retinoic acid and their derivatives, and hydroquinone, anti-wrinkle ingredients such as vitamin A and its derivatives, α-lipoic acid, adenosine, and α-hydroxy acids. Examples of the medicinal ingredients include acne treatment ingredients such as salicylic acid, and anti-inflammatory ingredients such as glycyrrhetinic acid (salt). The thin-film layer 20 may also contain a stabilizer such as dibutyl hydroxy toluene (BHT), vitamin E, or tocopherol acetate, which is a vitamin E derivative.

The functional substance may be a substance that enhances the UV blocking function of the thin-film layer 20. These functional substances are UV absorbers and UV scattering agents. Examples of the UV absorbers include octyl methoxycinnamate, octyl dimethoxybenzylidene dioxoimidazolidine propionate, hexyl diethyl aminohydroxy benzoylbenzoate, t-butyl methoxydibenzoylmethane, octyl triazone, and 2-ethylhexyl paramethoxycinnamate. Examples of the UV scattering agents include metal oxides such as titanium oxide and zinc oxide. When the thin-film layer 20 containing a UV absorber or UV scattering agent is attached to the transfer-receiving object, it is possible to protect the transfer-receiving object from the incidence of ultraviolet rays.

The functional substance may also be a substance that adds a decorative function to the thin-film layer 20 by coloring the thin-film layer 20. These functional substances are organic pigments and inorganic pigments that serve as coloring pigments.

The functional substance may also be a substance that adds a light scattering function to the thin-film layer 20. An example of such functional substance is particles. When the thin-film layer 20 contains particles, light scattering occurs inside the thin-film layer 20 due to the difference in refractive index between the particles and the substance surrounding them. The degree of such internal scattering varies depending on the particle shape and the difference in refractive index between the polymer material and the particles contained in the thin-film layer 20.

Examples of the particles include inorganic fillers and organic fillers. Materials for the inorganic filler include, for example, talc, silica, mica, alumina, zinc oxide, titanium oxide, barium sulfate, calcium carbonate, magnesium carbonate, barium silicate, calcium silicate, metallic soap, silicone, and the like. Materials for the organic filler include, for example, polyamide, polyethylene, polypropylene, acrylic resin, polyurethane, polytetrafluoroethylene, cellulose, and the like. In order to cause internal scattering in the thin-film layer 20, the particles may be made of a material having a refractive index that is different from that of the main component of the thin-film layer 20. The volume-average particle diameter of the particles contained in the thin-film layer 20 measured by the laser diffraction scattering method is preferably 0.5 µm or larger. In addition, the volume-average particle diameter is preferably 20.0 µm or less to reduce sedimentation of particles in the manufacturing process of the thin-film layer 20.

Note that, instead of adding particles to the thin-film layer 20, irregularities may be provided on the surface of the thin-film layer 20 to impart a light scattering function to the thin-film layer 20. The irregularities on the surface of the thin-film layer 20 cause the incident light to scatter at the surface. The degree of surface scattering varies depending on the shape of the irregularities on the surface of the thin-film layer 20 and the refractive index of the polymer material of the thin-film layer 20. The surface having the irregularities may be the first surface 21F or the second surface 21R, or may be both the first surface 21F and the second surface 21R. In the case where both the first surface 21F and the second surface 21R have irregularities, at the positions where convex portions are formed on the first surface 21F, there may be projections, recesses, or flat parts on the second surface 21R.

In the case when the thin-film layer 20 contains particles, and the particles exposed on the surface of the thin-film layer 20 form irregularities on the surface of the thin-film layer 20 that coincide with the shapes of the particles, in addition to the internal scattering, light scattering also occurs at the surface of the thin-film layer 20.

When the surface of the thin-film layer 20 has irregularities, it is possible to improve the anti-blocking properties, abrasion resistance, printability, and the like of the thin-film layer 20, in addition to the scattering function described above. In the case where the thin-film layer 20 is used for cosmetic purposes, and the cosmetic is applied from above the thin-film layer 20 after the thin-film layer 20 is attached to the skin, the irregularities on the second surface 21R enhance the adhesiveness of the cosmetics.

Examples of functions that may be improved or added by a functional substance in addition to the above-described functions include chemical resistance, oil resistance, weather resistance, moisture resistance, water resistance, rust prevention, antibacterial properties, and aromatic properties.

The thin-film layer 20 may be a single thin film or a laminate of thin films.

When the thin-film layer 20 includes a plurality of thin films, the plurality of thin films may include a thin film formed of the same material as other thin films or a thin film formed of materials different from other thin films.

When the thin-film layer 20 includes thin films made of different materials, the materials of the thin films may be selected so that the first surface 21F, which is to be attached to the transfer-receiving object, and the second surface 21R, which is exposed to the outside air after the thin-film layer 20 is transferred, are formed of materials that are suitable for such purposes of the surfaces. The thin films of the thin-film layer 20 may be arranged on the entire area or a partial area of the thin-film layer 20.

The support layer 30 includes a plurality of fibers. The support layer 30 may be, for example, a woven fabric, a knitted fabric, or a non-woven fabric. Of these examples, a non-woven fabric is preferred as the support layer 30. A non-woven fabric is composed of natural fibers or chemical fibers. Examples of the natural fibers that can be used include cotton, hemp, pulp, wool, and silk. Examples of the chemical fibers that can be used include polyester, polyolefin, cuprammonium rayon, rayon, lyocell, acetate, diacetate, nylon, aramid, and acrylic fibers. The non-woven fabric may be composed of fibers of different kinds, and they may include a natural fiber and a chemical fiber. Although the thickness of the support layer 30 is not particularly limited, for example, it may be selected to be in the range of 10 µm or more and 1 mm or less.

The method for manufacturing the non-woven fabric used as the support layer 30 is not specifically limited. The support layer 30 may be a non-woven fabric manufactured by one of the methods including spunlacing, spunbonding, needle-punching, melt blowing, air-laying, flash spinning, and resin bonding. The non-woven fabric used as the support layer 30 preferably has a basis weight in the range of 10 g/m² or more and 150 g/m² or less, and to improve its tactile feel, more preferably in the range of 20 g/m² or more and 50 g/m². The basis weight of the non-woven fabric refers to the average mass per unit area of the non-woven fabric.

If the support surface 31S of the support layer 30, which is the surface of the support layer in contact with the thin-film layer 20, has a plurality of fibers, a part of the support layer 30 may be made of a material other than fiber. For example, the support layer 30 may have a structure in which a non-woven fabric and a resin film are bonded together. When the support layer 30 is a laminate of a non-woven fabric and a resin film, the strength of the support layer 30 is increased as compared with the case where the support layer 30 is made of only a non-woven fabric.

### [Configuration of Contact Part Between Thin-Film Layer and Support Layer]

As described above, there are a plurality of fibers on the support surface 31S of the support layer 30, and the support surface 31S has irregularities formed by the fibers. Therefore, the second surface 21R of the thin-film layer 20 is not entirely in contact with the support surface 31S. Instead, the second surface 21R is partially in contact with the support surface 31S as it comes into contact with the parts where there are fibers. Since the thin-film layer 20 is partially in contact with the support layer 30, the force of adhesion between the thin-film layer 20 and the support layer 30 becomes smaller as compared with the case where the second surface 21R of the thin-film layer 20 is entirely in contact with the support layer 30. As a result, the support layer 30 can be easily peeled off from the thin-film layer 20 after bringing the transfer sheet 10 into contact with the transfer-receiving object.

Fig. 2 shows an example of the distribution of the parts where the thin-film layer 20 and the support layer 30 are in contact with each other in plan view of the transfer sheet 10 when a non-woven fabric is used as the support layer 30.

The thin-film layer 20 has a plurality of contact parts 22 in contact with the fibers of the support layer 30, and a non-contact part 23 separated from the fibers of the support layer 30. In Fig. 2, the contact parts 22 are shown dotted. Each contact part 22 extends elongated in the direction in which the fiber extends, and the arrangement of the contact parts 22 corresponds to the arrangement of the fibers at the support surface 31S. In the plan view, the plurality of contact parts 22 are dispersed, and the region between the contact parts 22 is the non-contact part 23.

The ratio of the area occupied by the contact parts 22 per unit area in the plan view is preferably in the range of 0.05% or more and 50% or less. When the ratio is in this range, the force of adhesion between the thin-film layer 20 and the support layer 30 is likely to be kept within a desirable range.

The thin-film layer 20 may not be in contact with all the fibers on the support surface 31S. A fiber may not be entirely in contact with the thin-film layer 20 in the direction in which the fiber extends. In other words, a fiber may be partially in contact with the thin-film layer 20 in the direction in which the fiber extends.

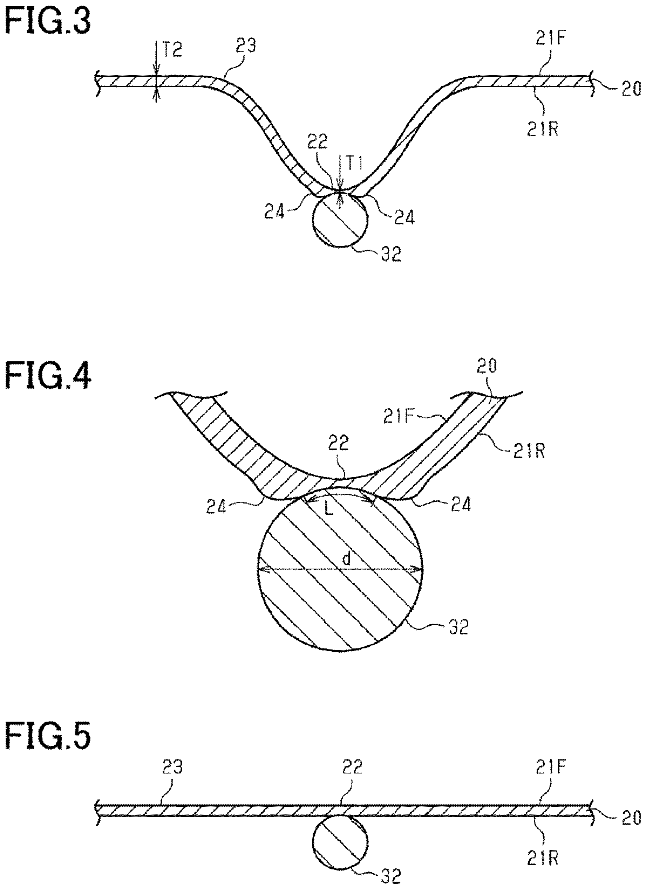

The structure of a contact part 22 and its vicinity in a cross section of the transfer sheet 10 will be described with reference to Figs. 3 and 4.

In the transfer sheet 10 of the present embodiment, as described above, since the thin-film layer 20 is partially in contact with the support layer 30, it is possible to prevent the adhesion between the thin-film layer 20 and the support layer 30 from becoming excessive. On the other hand, if the adhesion between the thin-film layer 20 and the support layer 30 is too small, it may be difficult to handle the transfer sheet 10 when the transfer sheet 10 is moved to the part of the transfer-receiving object where the thin-film layer 20 is to be attached, as the thin-film layer 20 may peel off from the support layer 30 or the thin-film layer may be wrinkled or twisted. In the present embodiment, the adhesion between the thin-film layer 20 and the support layer 30 is prevented from becoming too weak by controlling the state of adhesion between the thin-film layer 20 and the fibers at the contact parts 22.

As shown in Fig. 3, preferably, the thin-film layer 20 projects toward the support layer 30 at the contact parts 22. In other words, at the contacts parts 22, the first surface 21F has recesses that are recessed with respect to the surrounding parts, and the second surface 21R has projections that project from the surrounding parts.

When the thin-film layer 20 projects toward the support layer 30 at the contact parts 22, fibers 32 that could not have been in contact with the thin-film layer 20 if the thin-film layer 20 had been flat can be brought into contact. This makes it possible to increase the number of contact parts 22, and as a result, the adhesion between the thin-film layer 20 and the support layer 30 can be enhanced. In order to properly obtain such effects, the thin-film layer 20 preferably has at least one of the projecting contact parts, which is a contact part 22 projecting toward the support layer 30, per 1 mm² in plan view of the transfer sheet 10.

Note that a plurality of contact parts 22 may include at least one part where the contact parts 22 and their surrounding areas of the thin-film layer 20 are flat, as well as at least one part where the thin-film layer 20 spreads toward the support layer 30 and breaks near the contact parts 22. The contact parts 22 may also include parts where the thin-film layer 20 is deformed at the contact parts 22 such that it projects in the direction opposite to where the support layer 30 lies, that is, the thin-film layer 20 is pushed up by the fibers 32 at the contact parts 22 so that the first surface 21F has projections and the second surface 21R has recesses. Fig. 5 shows an example of a part of the thin-film layer 20 where the contact part 22 and its vicinity are flat.

The contact part 22 preferably extends along the surface of the fiber 32, that is, the contact part is in surface contact with the fiber 32. Specifically, the contact part 22 preferably extends along the circumference of the fiber 32. The thickness T1 of the thin-film layer 20 at the contact parts 22 is preferably less than the thickness T2 of the thin-film layer 20 at the non-contact part 23. That is, it is preferable that the contact parts 22 of the thin-film layer 20 are in close contact with the fibers 32 in such a manner that the thin-film layer 20 spreads thinly on the surfaces of the fibers 32. In this case, at each contact part 22, the second surface 21R of the thin-film layer 20 has a shape that matches the shape of the surface of the fiber 32. When the contact parts 22 have such configuration, the adhesion between the thin-film layer 20 and the support layer 30 at the contact parts 22 is increased as compared with the case where the contact parts 22 are in point contact with the fibers 32 when viewed in a cross section.

As shown in Fig. 4, specifically, it is preferable that the length L of a contact part 22 along the circumference of a fiber 32 is equal to or greater than 10% of the fiber diameter d of the fiber 32. In addition, it is preferable that 10% or more of the contact parts 22 of the thin-film layer 20 are in surface contact with the fibers 32, and each of these contact parts 22 has an average film thickness less than the average film thickness of the non-contact part 23 of the thin-film layer 20. According to such configuration, the adhesion between the thin-film layer 20 and the support layer 30 will not be too weak. Therefore, it is possible to prevent the thin-film layer 20 from peeling off from the support layer 30, or wrinkling or twisting of the thin-film layer when moving the transfer sheet 10 to the part of the transfer-receiving object where it should be attached.

The average film thickness of the contact parts 22 of the thin-film layer 20 is determined by cross-sectional observation using an electron microscope. For example, to determine the ratio of the contact parts 22 whose average film thickness is less than that of the non-contact part 23, the average film thickness is calculated for each of ten or more contact parts 22 included in a square region whose sides are 10 cm each in plan view of the transfer sheet 10. Specifically, samples for cross-sectional observation were prepared from a plurality of parts of the square region spread out on the region, and ten or more contact parts 22 were selected. For each contact part 22, the thickness T1 of the contact part 22 was measured at five measurement points equally spaced along the circumference of the fiber 32. The average of the thickness T1 values measured at the five points is the average film thickness of each contact part 22. The thickness T1 is the length of the thin-film layer 20 in the direction orthogonal to the tangent line of the first surface 21F at each measurement point.

The average film thickness of the non-contact part 23 of the thin-film layer 20 was obtained by averaging the average film thickness values of ten measurement areas set in the non-contact part 23 in the square region. The average film thickness of the measurement areas is determined by cross-sectional observation using an electron microscope. Specifically, samples for cross-sectional observation were prepared from a plurality of parts of the square region spread out on the region, and ten measurement areas were selected. For each measurement area, the thickness T2 of the non-contact part 23 was measured at ten measurement points equally spaced on the surface where the thin-film layer 20 extends. The average of the thickness T2 values at the ten measurement points is the average film thickness of each measurement area, and the average of the average film thicknesses values of the ten measurement areas is the average film thickness of the non-contact part 23. The thickness T2 is the length of the thin-film layer 20 in the direction orthogonal to the tangent line of the first surface 21F at each measurement point.

Since the thin-film layer 20 may have locally thin parts and locally thick parts, it is preferable that the measurement areas for obtaining the average film thickness of the non-contact part 23 are not set at such locally thin or thick parts. Specifically, the measurement areas preferably meet all of the following conditions (a), (b), and (c). (a) In the direction in which the thin-film layer 20 extends, a measurement area has a length equal to or greater than 1/2 of the circumferential length of the fiber 32 in contact with the contact part 22 closest to the measurement area. (b) The thin-film layer 20 is not in contact with a fiber 32 in the entire measurement area. (c) The coefficient of variation CV of the thickness T2 values at the ten measurement points of the measurement area is 0.15 or less.

The average film thickness of the non-contact part 23 is in the range of 1 nm or more and 5000 nm or less. Since the thin-film layer 20 is sufficiently thin when the average film thickness of the non-contact part 23 is within this range, the conformability of the thin-film layer 20 to the surface shape of the transfer-receiving object is enhanced, and as a result, the adhesion of the thin-film layer 20 to the transfer-receiving object can be enhanced. In order to further enhance the adhesion of the thin-film layer 20 to the transfer-receiving object, and, in the case when the transfer-receiving object is skin, to reduce the discomfort the living organism feels when the thin-film layer 20 is attached, the average film thickness of the non-contact part 23 is more preferably 1000 nm or less, and even more preferably 900 nm or less. To improve the strength the thin-film layer 20, the average film thickness of the non-contact part 23 is more preferably 100 nm or more, and even more preferably 300 nm or more.

Note that the contact parts 22 may include contact parts 22 whose average film thickness is equal to or greater than that of the non-contact part 23. For example, when a partially thick part is formed in the film formation of the thin-film layer 20, and the thick part comes into contact with a fiber 32, the average film thickness of the contact part 22 may be equal to or greater than the average film thickness of the non-contact part 23. When the contact parts 22 include at least one contact part 22 that is in surface contact with a fiber 32 and has an average film thickness less than that of the non-contact part 23, the adhesion between the thin-film layer 20 and the support layer 30 can be enhanced as compared with the case where such contact part 22 does not exist.

When the average film thickness converted from the mass of the thin-film layer 20 is assumed as a virtual film thickness, it is preferable that 10% or more of the contact parts 22 have an average film thickness less than the virtual film thickness. The virtual film thickness is the thickness of the film when it is assumed that a film having a uniform thickness is formed using all the materials of the thin-film layer 20, and is calculated based on the mass per unit area of the thin-film layer 20.

The length L of each contact part 22 along the circumference of the fiber 32 and the fiber diameter d are also obtained by cross-sectional observation as with the calculation of the average film thickness of the contact parts 22. For example, in the cross-sectional observation of ten or more contact parts 22 selected from the square region, the length L of each contact part 22 may be equal to or greater than 10% of the fiber diameter d of the fiber 32 in contact with the contact part 22.

The length L is the length of the part of the contact part 22 in contact with the fiber 32 along the circumference of the fiber 32 in a cross section taken along the thickness direction of the transfer sheet 10, and the fiber diameter d is the maximum diameter of the fiber 32 in the cross section.

The fiber diameter d of the fiber 32 is preferably 5 nm or more and 30 µm or less. When the fiber diameter is within this range, good transferability is likely to be ensured.

There is no upper limit to the length L, and the length L may exceed one half of the circumference of the fiber 32. However, more force is required to peel off the contact part 22 from the fiber 32 when the contact part 22 surrounds the fiber 32. Therefore, the length L is preferably equal to or smaller than 1/2 of the length of the circumference of the fiber 32.

The thin-film layer 20 preferably has a raised part 24 which is located adjacent to the contact parts 22. The raised parts 24 has a thickness locally increased relative to the rest of the contact parts 22. In other words, the raised parts 24 are parts that are raised compared to their surrounding areas of the thin-film layer 20. The adjacent area of the contact part 22 is an area having a length equal to or less than half the circumference of the fiber 32 along the extending direction of the thin film layer 20 from the center of the contact part 22 in the direction along the circumference of the fiber 32 in the cross section along the thickness direction of the transfer sheet 10. Even when the average film thickness of a contact part 22 is less than the average film thickness of the non-contact part 23, it is possible to prevent the strength of the thin-film layer 20 at the contact part 22 and around the contact part 22 from decreasing if the raised parts 24 are provided in the adjacent areas of the contact part 22.

Although an example where the raised parts 24 is formed on both sides of the contact part 22 is shown in Figs. 3 and 4, only one raised part 24 may be provided adjacent to one of the two ends of the contact part 22 along the direction in which the thin-film layer 20 extends. The plurality of contact parts 22 may include a contact part 22 which do not have a raised part 24 on either side of the contact part 22. The raised parts 24 belong to the non-contact part 23.

### [Method for Producing Transfer Sheet]

The transfer sheet 10 is formed by transferring the thin-film layer 20 formed on a film-formation substrate to the support layer 30.

The method of forming the thin-film layer 20 may be a thin-film formation method such as solution casting. In the solution casting method, the material for the thin-film layer 20 is dissolved or dispersed in a liquid to form a coating solution, and then the coating solution is applied to the film-formation substrate to form a coating film, and the coating film is dried to solidify the film, thereby forming the thin-film layer 20.

The coating solution can be applied to the film-formation substrate by known coating methods such as direct gravure, reverse gravure, small-diameter reverse gravure, Mayer coating, die, curtain, spray, spin coating, screen printing, comma, knife, gravure offset, and roll coating.

The thickness of the thin-film layer 20 to be formed is controlled by the proportion of the solid content contained in the coating solution and the coating method. In the case when the thin-film layer 20 is composed of a plurality of thin films, application of the coating solution and drying of the coating film are repeated to stack a plurality of thin films and form the thin-film layer 20. When thin films are partially formed, that is, when patterned thin films are formed, the coating solution may be applied by any suitable coating method such as direct gravure, spray, screen printing, or gravure offset.

Irregularities can be formed on the surface of the thin-film layer 20 by using a substrate having irregularities as the film-formation substrate, by pressing a plate having irregularities against the surface of the coating film before or after drying the coating film, or by patterning the thin film.

After forming the thin-film layer 20, the support surface 31S of the support layer 30 is brought into contact with the second surface 21R of the thin-film layer 20, which is the surface of the thin-film layer on the film-formation substrate, to transfer the thin-film layer 20 from the film-formation substrate to the support layer 30. Known transfer methods such as peeling by suction and a method using a sacrificial film can be used.

The transfer sheet 10 having the contact parts 22 and the non-contact part 23 described above can be formed by applying heat to the thin-film layer 20 and the support layer 30 while the thin-film layer 20 and the support layer 30 are in contact with each other. For example, the laminate of the thin-film layer 20 and the support layer 30 may be heated in an oven. The size, thickness, number, and the like of the contact parts 22 is controllable by adjusting the heating conditions. For example, heating conditions include heating temperature, heating time, and volume of hot air.

Specifically, the thin-film layer 20 softens by being heated, which causes the thin-film layer 20 to spread thinly and adhere tightly to the fibers 32, and also causes the raised parts 24 to be formed. The more softened the thin-film layer 20, the greater the number of the contact parts 22, the thinner the contact parts 22, and the greater the size of the contact parts 22. An increase in the size of the contact parts 22 means that the length L increases as a result of an increase in the contact area between the contact part 22 and the fiber 32. Further, the more softened the thin-film layer 20, the more likely the raised parts 24 are to be formed by the material that has moved from the contact parts 22 accumulating around the contact parts 22. The thin-film layer 20 may turn to liquid during heating if it is strongly softened.

The degree of softening of the thin-film layer 20 can be controlled by adjusting the heating temperature according to the relationship between the melting point of the material of the thin-film layer 20 and the heating temperature. The degree of softening of the thin-film layer 20 can also be controlled by adjusting the heating time. When the volume of hot air is large, the thin-film layer 20 is strongly pressed against the support layer 30, and thus the contact parts 22 extend thinly and the raised parts 24 are likely to be formed. Further, the more softened the thin-film layer 20 and the greater the volume of hot air, the more likely contact parts 22 having a shape projecting toward the support layer 30 to be formed.

The number and size of the contact parts 22 can also be changed by adjusting the film formation thickness of the thin-film layer 20. When the overall film thickness of the thin-film layer 20 is small, movement of the material caused by softening becomes limited, and therefore the contact parts 22 are less likely to be formed and the size of the contact parts 22 is likely to be small. The larger the film formation thickness of the thin-film layer 20, the greater the number of the contact parts 22 and the greater the size of the contact parts 22.

Based on these considerations, the film formation thickness of the thin-film layer 20 is preferably 100 nm or greater, and the average film thickness of the non-contact part 23 after forming the transfer sheet 10 is preferably 100 nm or more. When the film formation thickness is set so that the average film thickness of the non-contact part 23 becomes 100 nm or more, a desirable number and size of the contact parts 22 are likely to be obtained, and the adhesion between the thin-film layer 20 and the support layer 30 can be easily controlled.

### [Method of Transferring Thin-Film Layer]

When transferring the thin-film layer 20, the support layer 30 is peeled off from the thin-film layer 20 after bringing the first surface 21F of the thin-film layer 20 into contact with the part of the transfer-receiving object where it should be attached. When peeling off the support layer 30, a liquid may be supplied to the transfer sheet 10 on the transfer-receiving object so that the liquid permeates and reaches the boundary between the thin-film layer 20 and the support layer 30 to promote the peeling of the support layer 30 from the thin-film layer 20.

As described above, in the transfer sheet 10 of the present embodiment, the support surface 31S of the support layer 30 is made of fibers 32, and the state of the contact between the thin-film layer 20 and the fibers 32 is controlled so that the adhesion between the thin-film layer 20 and the support layer 30 is not too weak or strong. Thus, movement of the transfer sheet 10 to the target part and peeling off of the support layer 30 can be simply performed, leading to easy handling of the transfer sheet 10.

The transfer-receiving object is not particularly limited, and may be, for example, a living organism, a metallic product, a glass product, a resin product, a plant, paper, a solid medicine, or food. The transfer sheet 10 of this embodiment is particularly suitable when the transfer-receiving object is the skin or an organ of a living organism.

### [Examples]

The aforementioned transfer sheet will be described by way of specific examples. The materials used to form the transfer sheets of the following examples are as follows.
Film-formation substrate: Polyethylene-terephthalate film (Lumirror S10 manufactured by Toray Industries, Inc.)
Thin-film layer: DL-polylactic acid (weight average molecular weight: 440,000)
Support layer: Cellulose non-woven fabric (manufactured by Futamura Chemical Co., Ltd., basis weight: 20 g/m²)

### (Example 1)

A coating solution for the thin-film layer was prepared by dissolving the material of the thin-film layer in ethyl acetate so that the solid content in the solution became 3%. The coating solution for the thin-film layer was applied to the film-formation substrate using a wire bar (number #2). The coating film was solidified by drying the film, and the thin-film layer was obtained.

The support layer was placed on the thin-film layer on the film-formation substrate, and the thin-film layer was transferred from the film-formation substrate to the support layer. After transferring the thin-film layer, the laminate of the thin-film layer and the support layer was put into an oven, and heated for 20 seconds at a temperature of 60°C and an air velocity of 10 m/min. Thus, the transfer sheet of Example 1 was obtained.

Cross-sectional observation of the transfer sheet of Example 1 was performed using the method described later. As a result, the average film thickness of the non-contact part was 100 nm, the minimum length ratio, which is the ratio of the length Lof the contact part to the fiber diameter d having the smallest ratio among the 20 contact parts, was 5%, and the contact part thin-film proportion, which is the proportion of contact parts whose average thickness is less than that of the non-contact part among the 20 contact parts, was 5%.

### (Example 2)

A coating solution for the thin-film layer was prepared by dissolving the material of the thin-film layer in ethyl acetate so that the solid content in the solution became 3%. Using a wire bar (number #2), the coating solution for the thin-film layer was applied to a film-formation substrate coated with polyvinyl alcohol (PVA). The coating film was solidified by drying the film, and the thin-film layer was obtained.

To transfer the thin-film layer to the support layer, first, the laminate of the film-formation substrate and the thin-film layer was immersed in water so that the PVA dissolved and the thin-film layer peeled off from the film-formation substrate. Then, the thin-film layer was taken out of the water and placed on the support layer. After transferring the thin-film layer, the laminate of the thin-film layer and the support layer was put into an oven and heated for 30 seconds at a temperature of 80°C and an air velocity of 10 m/min. Thus, the transfer sheet of Example 2 was obtained.

Cross-sectional observation of the transfer sheet of Example 2 was performed using the method described later. As a result, the average film thickness of the non-contact part was found to be 100 nm, the minimum length ratio was 10%, and the contact part thin-film proportion was 10%.

### (Example 3)

A coating solution for the thin-film layer was prepared by dissolving the material of the thin-film layer in ethyl acetate so that the solid content in the solution became 5%. The coating solution for the thin-film layer was applied to the film-formation substrate using a wire bar (number #6). The coating film was solidified by drying, and the thin-film layer was obtained.

The support layer was placed on the thin-film layer on the film-formation substrate, and the thin-film layer was transferred from the film-formation substrate to the support layer. After transferring the thin-film layer, the laminate of the thin-film layer and the support layer was put into an oven and heated for 45 seconds at a temperature of 80°C and an air velocity of 10 m/min. Thus, the transfer sheet of Example 3 was obtained.

Cross-sectional observation of the transfer sheet of Example 3 was performed using the method described later. As a result, the average film thickness of the non-contact part was found to be 600 nm, the minimum length ratio was 12%, and the contact part thin-film proportion was 10%.

### (Example 4)

A coating solution for the thin-film layer was prepared by dissolving the material of the thin-film layer in ethyl acetate so that the solid content in the solution became 10%. The coating solution for the thin-film layer was applied to the film-formation substrate using a wire bar (number #3). The coating film was solidified by drying the film, and the thin-film layer was obtained.

The support layer was placed on the thin-film layer on the film-formation substrate, and the thin-film layer was transferred from the film-formation substrate to the support layer. After transferring the thin-film layer, the laminate of the thin-film layer and the support layer was put into an oven and heated for 45 seconds at a temperature of 80°C and an air velocity of 15 m/min. Thus, the transfer sheet of Example 4 was obtained.

Cross-sectional observation of the transfer sheet of Example 4 was performed using the method described later. As a result, the average film thickness of the non-contact part was found to be 600 nm, the minimum length ratio was 16%, and the contact part thin-film proportion was 15%.

### (Example 5)

A coating solution for the thin-film layer was prepared by dissolving the material of the thin-film layer in ethyl acetate so that the solid content in the solution became 5%. The coating solution for the thin-film layer was applied to the film-formation substrate using a wire bar (number #6). The coating film was solidified by drying, and the thin-film layer was obtained.

The support layer was placed on the thin-film layer on the film-formation substrate, and the thin-film layer was transferred from the film-formation substrate to the support layer. After transferring the thin-film layer, the laminate of the thin-film layer and the support layer was put into an oven and heated for 30 seconds at a temperature of 100°C and an air velocity of 15 m/min. Thus, the transfer sheet of Example 5 was obtained.

Cross-sectional observation of the transfer sheet of Example 5 was performed using the method described later. As a result, the average film thickness of the non-contact part was found to be 600 nm, the minimum length ratio was 16%, and the contact part thin-film proportion was 10%.

### (Example 6)

A coating solution for the thin-film layer was prepared by dissolving the material of the thin-film layer in ethyl acetate so that the solid content in the solution became 10%. The coating solution for the thin-film layer was applied to the film-formation substrate using a wire bar (number #3). The coating film was solidified by drying the film, and the thin-film layer was obtained.

The support layer was placed on the thin-film layer on the film-formation substrate, and the thin-film layer was transferred from the film-formation substrate to the support layer. After transferring the thin-film layer, the laminate of the thin-film layer and the support layer was put into an oven and heated for 45 seconds at a temperature of 100°C and an air velocity of 15 m/min. Thus, the transfer sheet of Example 6 was obtained.

Cross-sectional observation of the transfer sheet of Example 6 was performed using the method described later. As a result, the average film thickness of the non-contact part was found to be 600 nm, the minimum length ratio was 20%, and the contact part thin-film proportion was 15%.

### (Example 7)

A coating solution for the thin-film layer was prepared by dissolving the material of the thin-film layer in ethyl acetate so that the solid content in the solution became 10%. The coating solution for the thin-film layer was applied to the film-formation substrate using a wire bar (number #6). The coating film was solidified by drying, and the thin-film layer was obtained.

The support layer was placed on the thin-film layer on the film-formation substrate, and the thin-film layer was transferred from the film-formation substrate to the support layer. After transferring the thin-film layer, the laminate of the thin-film layer and the support layer was put into an oven and heated for 45 seconds at a temperature of 100°C and an air velocity of 20 m/min. Thus, the transfer sheet of Example 7 was obtained.

Cross-sectional observation of the transfer sheet of Example 7 was performed using the method described later. As a result, the average film thickness of the non-contact part was found to be 1200 nm, the minimum length ratio was 30%, and the contact part thin-film proportion was 20%.

### (Cross-sectional Observation)

The transfer sheet of each of the Examples was cut out into a square whose sides are 10 cm each. A spot for cross-sectional observation was randomly selected, and cross-sectional observation was performed using a scanning electron microscope. 20 contact parts were chosen from the observed region. For each contact part, the average film thickness and the ratio of the length L to the fiber diameter d were calculated, and also the presence or absence of a raised part adjacent to the contact part was confirmed. Further, ten measurement areas were set in the non-contact part of the observed region, and the average film thickness of the non-contact part was calculated.

### (Evaluation Method)

The transfer sheet of each Example was cut into a rectangle having a short side of 30 mm and a long side of 100 mm to prepare a test piece. As a friction test, the surface of the test piece on which the thin-film layer lies was rubbed back and forth three times with a load of 200 g/cm². The friction test was performed on three test pieces for each Example.

The results of the friction test were rated as follows: a rating "A" was given when no defects such as wrinkles, twists, or peeling could be seen in the thin-film layer of any of the three test pieces, a rating "B" was given when there were two test pieces that have no defects in the thin-film layer, and a rating "C" was given when there was only one test piece that has no defects in the thin-film layer. Regarding the Examples rated "B" and "C", the wrinkles, twists, and peeling in the test pieces such that the thin-film layers have been confirmed to have defects were all slight.

### (Evaluation Results)

For each of the Examples, the average film thickness of the non-contact part, the minimum value of the ratio of the length L to the fiber diameter d, the ratio of the contact parts whose average film thickness is less than that of the non-contact part, the presence or absence of the raised parts, and the evaluation result of the adhesion are shown in Table 1.

**[Table 1]**

| | Average film thickness of non-contact part | Length L of contact parts/fiber diameter | Proportion of contact parts with small average film thickness | Presence of raised parts | Adhesion |
|---|---|---|---|---|---|
| Example 1 | 100 nm | 5% | 5% | No | c |
| Example 2 | 100 nm | 10% | 10% | No | B |
| Example 3 | 600 nm | 12% | 10% | No | B |
| Example 4 | 600 nm | 16% | 15% | Yes | A |
| Example 5 | 600 nm | 16% | 10% | No | A |
| Example 6 | 600 nm | 20% | 15% | Yes | A |
| Example 7 | 1200 nm | 30% | 20% | No | A |

As shown in Table 1, every Example had a test piece that had no wrinkles, twists, or peeling in the thin-film layer. In particular, regarding Examples 2 to 7, whose ratio of length L to the fiber diameter d was 10% or greater and in which 10% or more of the contact parts had an average film thickness less than that of the non-contact part, no wrinkles, twists, and peeling were found in the thin-film layer in the majority of the test pieces, and good adhesion between the thin-film layer and the support layer was confirmed.

As described above in connection with the embodiments and Examples, according to the transfer sheet, the advantages listed below can be achieved.
(1) The thin-film layer 20 has a plurality of contact parts 22 in contact with the fibers 32 in the support layer 30, and a non-contact part 23 separated from the fibers 32. The plurality of contact parts 22 include a contact part 22 that is in surface contact with the fiber 32 and has an average film thickness less than that of the non-contact part 23.
   According to this configuration, since the thin-film layer 20 is partially in contact with the support layer 30, it is possible to prevent the adhesion between the thin-film layer 20 and the support layer 30 from becoming excessive. As a result, the support layer 30 can be easily peeled off from the thin-film layer 20 after bringing the transfer sheet 10 into contact with the transfer-receiving object. On the other hand, since at least a part of the plurality of contact parts 22 are in contact with the fibers 32 in such a manner that the thin-film layer 20 spreads thinly on the surfaces of the fibers 32 of the support layer 30, it is possible to prevent the adhesion between the thin-film layer 20 and the support layer 30 from becoming too weak. As a result, it is possible to prevent the thin-film layer 20 from peeling off from the support layer 30, or wrinkling or twisting of the thin-film layer 20 when moving the transfer sheet 10 to the transfer-receiving object. Therefore, the transfer sheet 10 can be easily handled.
(2) The contact part 22 that is in surface contact with the fiber 32 has the average film thickness less than that of the non-contact part 23, and has a length along the circumference of the fiber 32 equal to or greater than 10% of the fiber diameter d of the fiber 32. According to this configuration, the contact part 22 is in surface contact with the fiber 32, and the area of the part in contact with the fiber 32 are large enough to advantageously prevent the adhesion between the thin-film layer 20 and the support layer 30 from becoming too small.
(3) The thin-film layer 20 has a raised part 24 which is located adjacent to at least a part of the contact parts 22. The raised part 24 has a thickness locally increased relative to the rest of the contact parts 22. According to this configuration, even when the average film thickness of a contact part 22 is less than the average film thickness of the non-contact part 23, it is possible to prevent the strength of the thin-film layer 20 at the contact part 22 and around the contact part 22 from decreasing.
(4) The plurality of contact parts 22 include a projecting contact part where the thin-film layer 20 projects toward the support layer 30. According to this configuration, fibers 32 that could not have been in contact with the thin-film layer 20 if the thin-film layer 20 had been flat can be brought into contact. This makes it possible to increase the number of contact parts 22, and thus the adhesion between the thin-film layer 20 and the support layer 30 can be enhanced. Further, when the thin-film layer 20 has one or more of the projecting contact parts per 1 mm² in plan view of the transfer sheet 10, the effect of enhancing the adhesion between the thin-film layer 20 and the support layer 30 is favorably obtained.

## Claims

1. A transfer sheet comprising:
a thin-film layer having an average mass of 0.01 g/m² or more and 10 g/m² or less; and
a support layer that supports the thin-film layer, wherein
the support layer has a support surface in contact with the thin-film layer, the support surface includes a plurality of fibers,
the thin-film layer includes a plurality of contact parts in contact with the fibers and a non-contact part separated from the fibers, and
the plurality of contact parts include at least one of the contact parts that is in contact with at least one of the fibers such that the at least one of the contact parts extends along a surface of at least one of the fibers in contact therewith, an average film thickness of the thin-film layer at the at least one of the contact parts is less than an average film thickness of the thin-film layer at the non-contact part.

2. The transfer sheet according to claim 1, wherein the at least one of the contact parts is in contact with the one of the fibers so as to extend along the surface of the one of the fibers, the at least one of the contact parts having the average film thickness less than the average film thickness of the non-contact part, the at least one of the contact parts having a length along a circumference of the one of the fibers equal to or greater than 10% of a fiber diameter of the one of the fibers.

3. The transfer sheet according to claim 1 or 2, wherein the thin-film layer has a raised part which is located adjacent to at least a part of the contact parts, the raised part having a thickness locally increased relative to a rest of the contact parts.

4. The transfer sheet according to any one of claims 1 to 3, wherein the plurality of contact parts include at least one of projecting contact parts where the thin-film layer projects toward the support layer.

5. The transfer sheet according to claim 4, wherein the thin-film layer has one or more of the projecting contact parts per 1 mm² in plan view of the transfer sheet.
